# EUROPÄISCHE PATENTSCHRIFT

(11) **EP 1 626 661 B1**
(45) Veröffentlichungstag und Bekanntmachung des Hinweises auf die Patenterteilung: **25.11.2009**
(21) Anmeldenummer: 04734218.3
(22) Anmeldetag: 21.05.2004
(51) Int. Cl.: A61B 17/02

(54) **RETRAKTOR ZUR DURCHFÜHRUNG VON HERZ- UND THORAXCHIRURGISCHEN EINGRIFFEN**
RETRACTOR FOR PERFORMING HEART AND THORAX SURGERIES
ECARTEUR PERMETTANT D'EFFECTUER DES INTERVENTIONS CHIRURGICALES CARDIAQUES ET THORACIQUES

(30) Priorität: 28.05.2003 DE 10325393
(43) Veröffentlichungstag der Anmeldung: 22.02.2006
(73) Patentinhaber: Karl Storz GmbH & Co. KG, 78532 Tuttlingen (DE)
(72) Erfinder: SCHÖLLHORN, Joachim, 79104 Freiburg (DE)
(74) Vertreter: Weller, Wolfgang
(86) Internationale Anmeldenummer: PCT/EP2004/005468
(87) Internationale Veröffentlichungsnummer: WO 2004/105619

(56) Entgegenhaltungen:
- DE-U- 20 003 335
- US-A- 5 967 972
- US-B1- 6 478 734

## Beschreibung

Die Erfindung betrifft einen Retraktor mit einer Schiene, von der ein erster Haltearm abgewinkelt absteht, sowie mit einem zweiten Haltearm, der sich etwa parallel zum ersten Haltearm erstreckt und wobei deren Abstand veränderbar ist, und mit an den Haltearmen angebrachten Funktionselementen, von denen zumindest ein Funktionselement einen sich quer zu einem Haltearm erstreckenden Abschnitt aufweist, der um eine Achse, die etwa parallel und im Abstand zu einem Haltearm verläuft verschwenkbar ist, wobei der Abschnitt mit einem Schwenkbügel um die Achse schwenkbar verbunden ist, wobei der Schwenkbügel fest mit dem Haltearm verbunden ist.

Ein derartiger Rektraktor ist aus der US-A-5,025,779 oder der DE-U-200 03 335 bekannt.

Retraktoren dieser Art werden bspw. als Rippensperrer bei Operationen am offenen Herzen eingesetzt. Es haben sich hierbei die Fachbegriffe Thorax- und Sternumsperrer für diese Retraktoren etabliert.

Bei der Verwendung als Sternumsperrer wird das Sternum (Brustbein) der Länge nach aufgetrennt und die jeweils an den beiden Haltearmen befestigten Funktionselemente in die Öffnung des Sternums eingeführt. Durch Betätigen des Antriebsmechanismus werden die beiden Haltearme von einander wegbewegt und der Brustkorb gespreizt, hierdurch wird ein Eingriff in die Brusthöhle durch den Operateur ermöglicht.

Sternumsperrer werden vor allem bei Operationen verwendet, bei denen Herzkranzgefäße durch andere Gefäße ersetzt werden, sogenannte Bypass-Operationen. Hierzu wird heutzutage im Allgemeinen die linke Brustbeinarterie hinzugezogen. Diese wird in der Fachsprache häufig als (Left) Internal Mammary Artery oder abgekürzt (L)IMA bezeichnet. Daher werden Retraktoren für diese Art von Operation auch als IMA- oder LIMA-Sperrer bezeichnet.

Eine Weiterentwicklung dieser Operationstechnik ist die sogenannte MIDCAB(= minimal invasive direct coronar artery bypass)-Technik. Diese Technik wurde im Rahmen der minimalinvasiven Chirurgie entwickelt. Hierbei erfolgt der Zugang zum Herzen nicht durch das Öffnen des Brustbeins, sondern über einen Zwischenrippenraum. Die hierzu entwickelten Geräte werden in der Fachsprache als MIDCAB-Sperrer oder manchmal auch als Lateralsperrer bezeichnet.

Für beide Techniken hat es sich als nützlich erwiesen, wenn die dabei verwendeten Sperrer nicht nur die beiden Seiten der Operationsöffnung auseinander ziehen, sondern auch die herzseitige Hälfte des Brustkorbs gegenüber der anderen anheben. Hierdurch wird ein besserer Zugang zum Herzen ermöglicht.

Das eingangs erwähnte Dokument US-A-5,025,779 beschreibt dazu einen Rippensperrer zum Anheben der einen Seite der Operationsöffnung im Vergleich zu der anderen Seite. Die in dem Dokument beschriebene Vorrichtung verfügt über zumindest ein Funktionselement, das um eine Achse parallel zu einem Haltearm schwenkbar gelagert ist, und dessen Schwenkstellung zu der Retraktorschiene mit Hilfe eines Stellorgans verändert werden kann.

Bei diesem Stellorgan handelt es sich um eine Stellschraube. Diese Stellschraube erstreckt sich durch einen Schwenkbügel, mit dem das Funktionselement auf dem Haltearm angebracht ist, und stützt sich gegen den Abschnitt des Funktionselements ab, der sich quer zum Haltearm erstreckt. Durch Drehen der Stellschraube wird der Abstand zwischen Schwenkbügel und Funktionselement auf der Höhe der Stellschraube verändert. Dadurch, dass der Schwenkbügel und das Funktionselement an einer zweiten Stelle schwenkbar miteinander verbunden sind, kommt es zu der gewünschten Änderung der Schwenkstellung.

Die Verwendung eines solchen Stellorgans birgt allerdings einige Nachteile. Ein Nachteil einer solchen Konstruktion ist, dass der Operateur kein genaues Gefühl dafür hat, wie viel Kraft auf den Körper des Patienten ausgeübt wird, da die Veränderung der Schwenkstellung wie oben Beschrieben durch Betätigen einer Stellschraube erfolgt.

Die auf den Körper des Patienten ausgeübte Kraft wirkt hierbei in Richtung der Achse des Stellorgans. Die Kraft wird teilweise durch das Stellorgan und teilweise durch die Verbindung zwischen Schwenkbügel und Funktionselement aufgenommen. Das einzige Gefühl, das der Operateur für die ausgeübte Kraft hat, ist hierbei die Schwergängigkeit der Stellschraube. Durch eine zu hohe Krafteinwirkung kann es zu Traumata im Bereich der Operationsöffnung kommen.

Ein solches Stellorgan übt außerdem eine relativ hohe punktuelle Belastung auf das Funktionselement aus. Das Sperren eines Brustkorbs ist, da dieser durch zahlreiche Knochen, Muskeln und Sehnen aufgebaut ist, mit einem hohen Kraftaufwand verbunden.

Diese hohe Kraft wirkt daher direkt auf die relativ kleine Auflagefläche des Stellorgans ein.

Außerdem ist das Anheben einer Brusthälfte mit Hilfe einer Stellschraube zeitaufwendig. Um sowohl eine genügende Genauigkeit der Einstellung als auch eine Leichtgängigkeit der Stellschraube zu gewährleisten, werden Stellschrauben mit einem engen Gewinde mit geringer Steigung verwendet. Dadurch ist die Veränderung der Schwenkstellung pro Umdrehung der Schraube gering, was bei einer größeren Veränderung der Schwenkstellung ein langwieriges Drehen notwendig macht.

Aus der US-B-6 478 734 ist ein Rektraktor mit einer Schiene bekannt, von der ein erster Haltearm abgewinkelt absteht, sowie mit einem zweiten Haltearm, der sich etwa parallel zum ersten Haltearm erstreckt und deren Abstand veränderbar ist, und mit an den Haltearmen angebrachten Funktionselementen, von denen zumindest ein Funktionselement einen sich quer zu einem Haltearm erstreckenden Abschnitt aufweist, der um eine Achse in etwa parallel zu einem Haltearm verschwenkbar ist, wobei die jeweilige Schwenkstellung des Funktionselements durch Ergreifen mit einer Hand einstellbar ist und durch einen Riegelmechanismus fixierbar ist. Die Funktionselemente werden indirekt über die Verstellung der beiden Haltearme aktuiert. Dafür ist das Funktionselement bezüglich dem Haltearm, an dem es angebracht ist, frei drehbar, und zwar um dessen Längsachse.

Das Dokument US-A-6,159,231 beschreibt einen Retraktor, bei dem die Schiene, auf der die beiden Haltearme angebracht sind, abgewinkelt ist. Das Anheben des Retraktors erfolgt hierbei durch einen weiteren äußeren Mechanismus, z.B. einen Seilzug.

Diese Vorrichtung hat allerdings den Nachteil, dass sie den Zugang zur Operationsöffnung für den Operateur deutlich vermindert. So kann bei dieser Ausführung der Zugang zur Operationsöffnung nur von der dem Mechanismus zum Anheben entfernten Seite erfolgen. Des Weiteren ist die in dem Patent beschriebene Vorrichtung nicht symmetrisch, kann also nur für eine, im Allgemeinen die linke, Brusthälfte verwendet werden.

Das Dokument DE-C-198 57 320 beschreibt einen Rippensperrer, dessen Schiene, auf der die beiden Haltearme befestigt sind, konvex bogenförmig ist. Der beschriebene Retraktor verfügt hierbei über keinerlei weitere Elemente zum Anheben der einen Seite der Operationsöffnung gegenüber der anderen. Dadurch ist die Höhendifferenz zwischen den beiden Seiten der Operationsöffnung direkt von der Öffnungsweite abhängig. Dies wird allein von der festen Krümmung der bogenförmigen Schiene bestimmt. Dies bedeutet, dass der Retraktor nicht individuell auf die Gegebenheiten einer Operation angepasst werden kann.

Das Dokument US-A-6,416,468 beschreibt einen lateralen Rippensperrer, der einen an einem der beiden Haltearme befestigten Fuß aufweist. Die in diesem Patent beschriebene Vorrichtung ist mechanisch kompliziert und besteht aus einer großen Anzahl von Einzelteilen. Dies macht diese Vorrichtung in der Herstellung kostspielig und kompliziert zu reinigen und sterilisieren. Außerdem kann es durch die Verwendung eines separaten Fußes zum Abrutschen desselben und damit zu einer Störung der Operation kommen.

Es ist daher Aufgabe der Erfindung, einen Retraktor zum Anheben der Rippen zu schaffen, bei dem das Anheben der Brusthälfte atraumatisch und vom Operateur manuell fühlbar durchführbar ist.

Die Aufgabe wird dadurch gelöst, dass die jeweilige Schwenkstellung des Funktionselements relativ zum Schwenkbügel und somit zum Haltearm durch Ergreifen mit einer Hand einstellbar ist und durch einen Riegelmechanismus fixierbar ist.

Durch das Einstellen der Schwenkstellung durch Anheben von Hand hat der Operateur jederzeit ein Gefühl dafür, wie viel Kraft auf den Brustkorb des Patienten ausgeübt wird, und kann dadurch Traumata durch Überbelastung vermeiden. Ist die vom Operateur gewünschte Verschwenkstellung erreicht, wird diese durch den Riegelmechanismus fixiert.

Der Riegelmechanismus ist hierbei vorteilhafterweise mechanisch einfach ausgeführt. Es kann sich hierbei zum Beispiel um ein Rastensystem, ein System, das mit Stiften gesichert wird, oder um andere dem Fachmann bekannte Mittel handeln.

In einer bevorzugten Ausführungsform ist der Riegelmechanismus als Rastenmechanismus ausgebildet.

Ein Rastenmechanismus erweist sich als besonders vorteilhafter Riegelmechanismus, da durch diesen die Einstellung der Schwenkstellung besonders einfach und feinstufig ist und durch die Verwendung eines Rastenmechanismus außerdem ein Abrutschen während der Fixierung der Schwenkstellung vermieden werden kann.

Die Fixierung der Schwenkstellung mittels des Rastenmechanismus kann mit nur einer Hand erfolgen. Die Raste ist zweckmäßigerweise in Richtung des Anhebens einer Brusthälfte beweglich und blockiert in Richtung des Absenkens der Brusthälfte. Damit kann die Brusthälfte durch einfaches Anheben in die gewünschte Stellung gebracht werden und durch kurzes Absenken mit einer Hand in der gewünschten Stellung verriegelt werden.

In einer bevorzugten Ausgestaltung der zuvor genannten Maßnahme wird der Rastenmechanismus aus einer Zahnstange und einer Rastnase gebildet.

Ein solcher Rastenmechanismus zeichnet sich durch seine besondere mechanische Einfachheit aus, da er aus wenigen Teilen gebildet werden kann und keine weiteren beweglichen Teile aufweist, was sowohl die Fertigung als auch die Reinigung und Sterilisation des Retraktors erheblich vereinfacht.

In einer weiteren bevorzugten Ausgestaltung der oben genannten Maßnahme wird die Rastnase durch eine Aussparung im Funktionselement gebildet.

Die Ausbildung der Rastnase durch eine Aussparung im Funktionselement erleichtert die Fertigung des Funktionselements deutlich. Außerdem werden hierbei Ecken und Nischen vermieden, in denen sich Bakterien einnisten können und die schwer zu reinigen und zu sterilisieren sind.

In einer weiteren bevorzugten Ausführungsform ist die Rastnase an der der Zahnstange zugewandten Seite abgeschrägt.

Durch diese Maßnahme wird ein besonders festes Eingreifen der Rastnase in die Zahnstange sichergestellt

In einer weiteren bevorzugten Ausführungsform erstreckt sich die Zahnstange in in etwa rechtem Winkel zur Längsachse des Haltearms von dem Retraktor weg.

Durch eine Ausrichtung der Zahnstange in dieser Richtung weist diese von der Operationsöffnung weg und vermeidet eine Behinderung des Operateurs. Außerdem wird die Gefahr eines ungewollten Bewegens der Zahnstange minimiert.

In einer weiteren bevorzugten Ausführungsform weist der Riegelmechanismus einen T-förmigen Griff auf.

Die Verwendung eines T-förmigen Griffes erleichtert die Bedienung des Riegelmechanismus erheblich. Ein T-förmiger Griff erleichtert außerdem die einhändige Fixierung der Schwenkstellung, da dieser, wenn das Funktionselement mit der Hand ergriffen wird, mit dem Daumen bedient werden kann.

In einer weiteren bevorzugten Ausführungsform kann das Funktionselement frei auf dem Haltearm verschoben werden.

Durch die freie Verschiebbarkeit des Funktionselements kann der Retraktor an die jeweilige Anatomie des Patienten individuell angepasst werden.

In einer weiteren bevorzugten Ausgestaltung der oben genannten Maßnahme ist das Funktionselement vom Haltearm abnehmbar.

Die Abnehmbarkeit des Funktionselements vom Haltearm erleichtert die Reinigung und Sterilisation des Retraktors deutlich.

In einer weiteren bevorzugten Ausgestaltung der oben genannten Maßnahme kann das Funktionselement an jeden der Haltearme angebracht werden.

Diese Maßnahme erhöht die Flexibilität des Retraktors deutlich. Damit kann der Retraktor sowohl bei Operationen auf der linken als auch auf der rechten Brustseite eingesetzt werden, als auch sowohl als Lateralsperrer wie auch als Sternumsperrer. Außerdem gibt es dem Operateur deutlich mehr Freiheit auszuwählen, wie der Retraktor auf dem Patienten aufliegt.

In einer weiteren bevorzugten Ausführungsform ist das Funktionselement an den Flächenbereichen, mit denen es am Rande einer Operationsöffnung oder im Körper anliegt, zumindest teilweise mit einer konturierten Oberfläche versehen.

Diese Maßnahme hat den Vorteil, dass durch die strukturierte Oberfläche die Fixierung zwischen Funktionselement und Körpergewebe gefördert wird. Dadurch können am Retraktor Manipulationen durchgeführt werden, ohne dass dabei der lagefeste Eingriff zwischen dem Funktionselement und dem Gewebe verändert wird.

In einer weiteren bevorzugten Ausgestaltung der oben genannten Maßnahme ist die Oberflächenstruktur gekreuzt fischgrätenartig ausgebildet.

Diese Maßnahme hat den Vorteil, dass sie nicht nur fertigungstechnisch gut hergestellt werden kann, sondern auch in unterschiedliche Raumrichtungen einen unverschieblichen und atraumatischen Eingriff mit dem Gewebe ermöglicht.

In einer weiteren bevorzugten Ausführungsform weist die Oberflächenstruktur pyramidenförmige Erhebungen mit rautenförmiger Basis auf.

Diese Maßnahme hat den Vorteil, dass durch die Rautenstruktur der Oberflächenriffelung in zwei unterschiedliche zueinander senkrechte Raumrichtungen unterschiedlich starke Widerstände gegen Verschieben geschaffen werden können. So ist ein Verschieben in Richtung der längeren Diagonale eher möglich als in Richtung der kürzeren Diagonale, da in dieser Richtung die den Widerstand bietenden Erhebungen deutlich breiter sind. Außerdem befinden sich in dieser Richtung deutlich mehr Erhebungen pro Längeneinheit.

Vorteilhafterweise ist an einem Haltearm das Funktionselement und am anderen eine Valve angebracht.

In dieser Ausgestaltung ist der Retraktor besonders für die Durchführung von herz- und thoraxchirurgischen Eingriffen geeignet.

Es versteht sich, dass die vorstehend genannten und die nachstehend noch zu erläuternden Merkmale nicht nur in der jeweilig angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:
- Fig. 1: eine perspektivische Ansicht eines erfindungsgemäßen Retraktors mit einem erfindungsgemäßen Funktionsele- ment,
- Fig. 2: eine perspektivische Ansicht eines vom Retraktor abgenommenen erfindungsgemäßen Funktionselements in hochgeschwenktem Zustand,
- Fig. 3: einen Schnitt durch ein erfindungsgemäßes Funktions- element längs der Linie IV-IV von Fig. 2,
- Fig. 4: eine Vergrößerung des umkreisten Ausschnittes von Fig. 3,
- Fig. 5: einen Querschnitt eines menschlichen Brustkorbes, in den ein Retraktor eingesetzt ist,
- Fig. 6: eine der Schnittdarstellung von Fig. 5 entsprechende Darstellung mit gespreiztem Retraktor, und
- Fig. 7: eine den Figuren 5 und 6 entsprechende Darstellung mit angehobener Brusthälfte, kurz vor dem Fixieren durch den Riegelmechanismus.

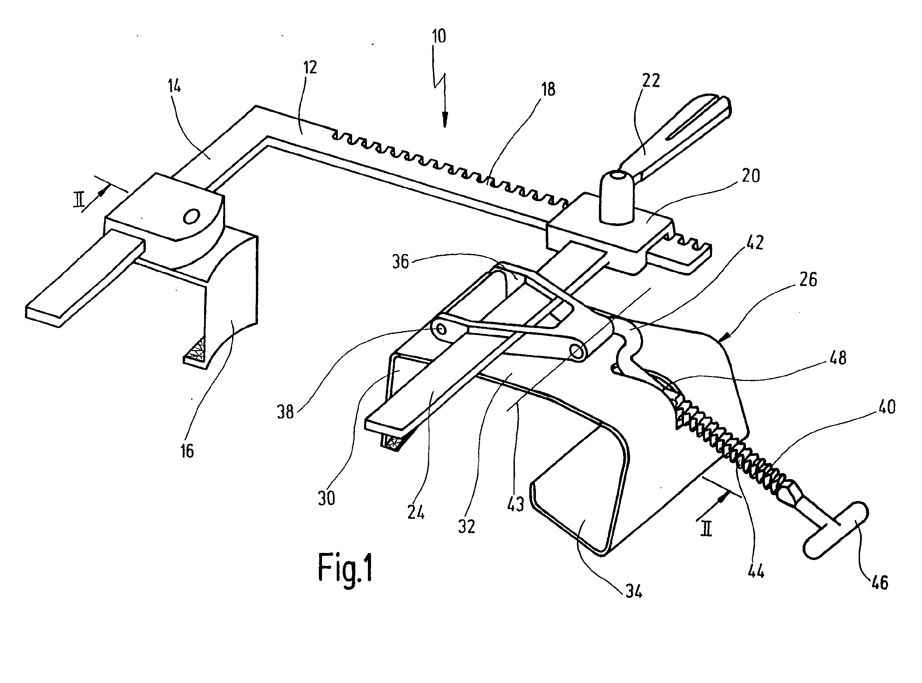

In Fig. 1 ist ein erfindungsgemäßer Retraktor in seiner Gesamtheit mit der Bezugsziffer 10 bezeichnet.

Der Retraktor 10 weist eine Schiene 12 auf, zu der rechtwinklig ein Haltearm 14 angebracht ist. Auf diesem Haltearm 14 ist eine Valve 16 verschiebbar angebracht.

Die Schiene 12 weist außerdem einen Zahnstangenabschnitt 18 auf, auf dem ein Antriebsmechanismus 20 verschiebbar angebracht ist. Das Verschieben des Antriebsmechanismus 20 erfolgt durch Eingreifen eines in der Zeichnung nicht dargestellten Zahnrades in die Zähne des Zahnstangenabschnitts 18. Dieses Zahnrad wird mit Hilfe einer Kurbel 22 betätigt.

An dem Antriebsmechanismus 20 ist außerdem ein zweiter Haltearm 24 angebracht, der in etwa in rechtem Winkel zur Schiene 12 steht. Auf diesem Haltearm 24 ist ein Funktionselement 26 verschiebbar angebracht.

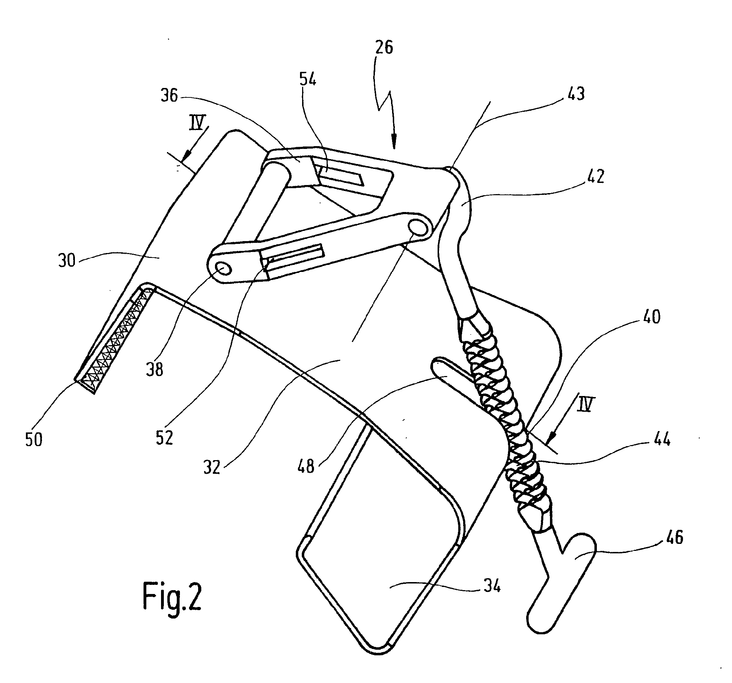

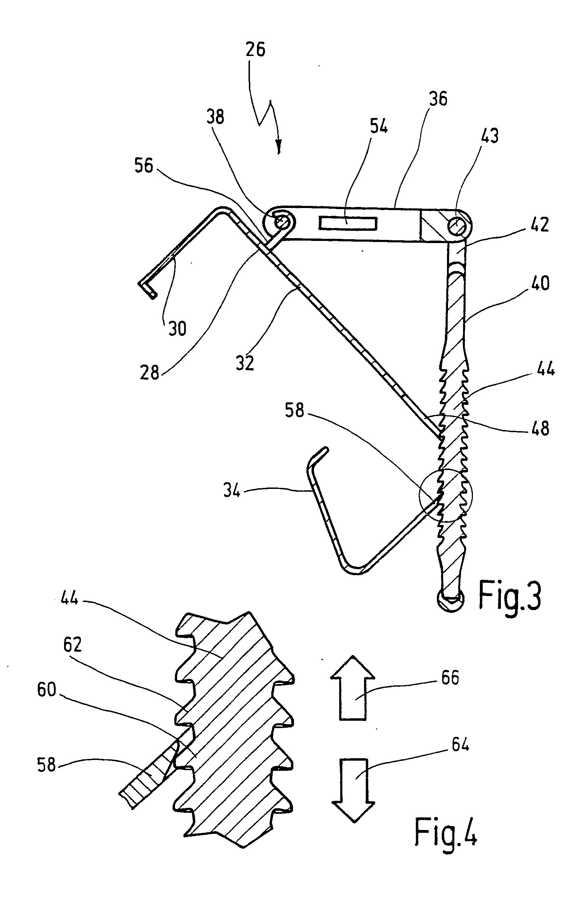

Das Funktionselement 26 (siehe auch Figuren 2 und 3) weist einen Valvenabschnitt 30, der in etwa parallel zur Valve 16 steht, einen Querabschnitt 32, der sich quer zum Haltearm 24 erstreckt, und einen abstützenden Abschnitt 34 auf. Der Querabschnitt 32 ist schwenkbar mit einem Schwenkbügel 36 verbunden. Der Schwenkbügel 36 weist dazu an einem Ende einen Achszapfen 38 auf, in den das Funktionselement 26 eingehängt ist.

Dazu steht von der Oberseite des Querabschnittes 32 ein entsprechend geformter Haken 56 hoch.

Der Schwenkbügel 36 weist zwei Öffnungen 52 und 54 auf (siehe insbesondere Fig. 2), durch die ein Haltearm 24 hindurchschiebbar ist (siehe insbesondere Fig. 1). Dadurch ist das Funktionselement 26 längsverschieblich am Retraktor 10 angebracht.

Der Schwenkbügel 36 ist an dem dem Achszapfen 38 gegenüberliegenden Ende mit einem Stellorgan 40 verbunden. Dieses Stellorgan weist einen U-förmigen hakenartigen Abschnitt 42 auf, der mit dem Schwenkbügel 36 verbunden ist. Der Endabschnitt des U-förmigen Abschnitts 42 ist dabei in eine Öffnung im Schwenkbügel 36 seitlich eingesteckt und um eine Achse 43, die etwa parallel zum Haltearm 14 verläuft, verschwenkbar. Das Stellorgan 40 ist somit schwenkbar mit dem Schwenkbügel 36 verbunden.

Das Stellorgan 40 weist außerdem eine Zahnstange 44 auf, die sich in etwa in rechtem Winkel zum Haltearm 24 erstreckt und somit sich von dem Retraktor 10 weg erstreckt. Am Ende der Zahnstange 44 ist ein T-förmiger Griff 46 angebracht.

Die Zahnstange 44 greift in dieser Darstellung in einer Aussparung 48 des Funktionselements 26 ein. Diese Aussparung 48 ist schlitzförmig und erstreckt sich im Übergangsbereich von Querabschnitt 32 zum abstützenden Abschnitt 34.

Fig. 2 zeigt ein vom Retraktor 10 abgenommenes Funktionselement 26, wobei Querabschnitt 32 und Schwenkbügel 36 relativ zueinander verschwenkt sind.

In dieser Darstellung wird sichtbar, dass der Valvenabschnitt 30 des Funktionselements 26 an seiner Innenseite ein rautenförmiges Muster 50 aufweist.

In dieser Darstellung wurde der Querabschnitt 32 gegenüber dem Schwenkbügel 36 um den Achszapfen 38 verschwenkt. Der hakenförmige Abschnitt 42 des Stellorgan 40 wurde hierbei gegenüber dem Querabschnitt 32 angehoben. Das Stellorgan 40 wurde gegenüber dem Schwenkbügel 36 um die Achse 43 geschwenkt, wobei die Zahnstange 40 wiederum in der Aussparung 48 zum Liegen kommt.

In Fig. 3 wird der Haken 56 sichtbar, der am Querabschnitt 32 angebracht ist.

Ebenfalls ist ersichtlich, dass die untere Kante der Aussparung 48 im Bereich des abstützenden Abschnitts 34 des Funktionselements 26 eine Rastnase 58 bildet, die hier an der Zahnstange 44 anliegt.

Fig. 4 zeigt einen vergrößerten Ausschnitt aus Fig. 3, wobei sichtbar wird, wie die Rastnase 58 an einem ersten Zahn 60 der Zahnstange 44 anliegt. Es ist hier ebenfalls sichtbar, dass die Rastnase 58 abgeschrägt ist. Die Abschrägung steht in Berührung mit einer schrägen Flanke des Zahnes 60.

Aus dieser Figur geht hervor, dass die Rastnase 58 bei einer Bewegung in Richtung des Pfeils 64 an dem ersten Zahn 60 vorbeigleiten kann und damit in dieser Richtung frei beweglich ist. Bei der entgegengesetzten Bewegung in Richtung des Pfeils 66 stößt die Rastnase gegen eine rechtwinklig zur Längsachse der Zahnstange 44 verlaufende Flanke des Zahnes 62, wodurch eine Bewegung in Richtung des Pfeils 66 blockiert ist.

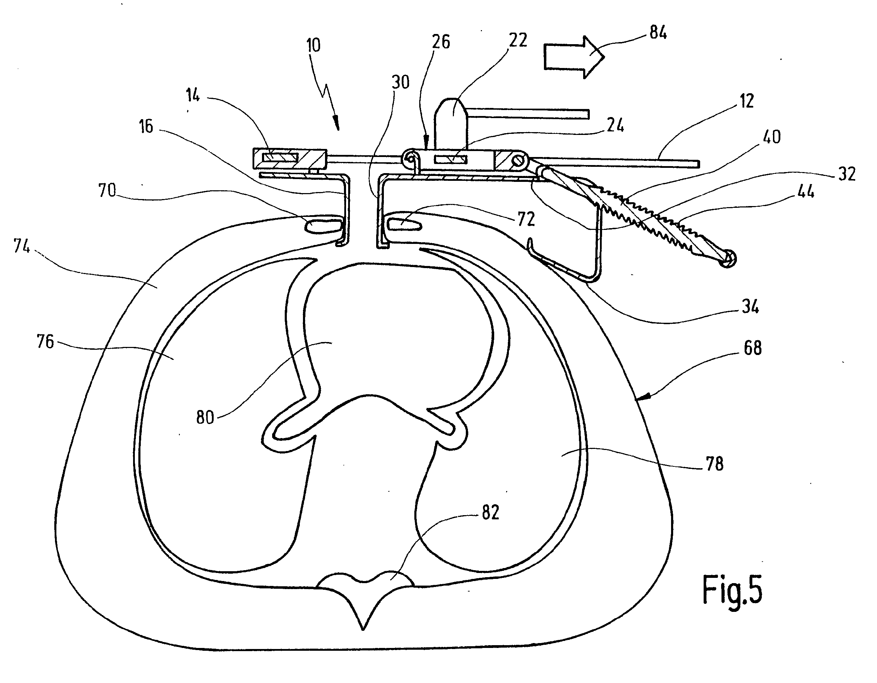

Fig. 5 zeigt die schematische Darstellung eines Schnitts durch einen Körper 68 eines Patienten, wobei ein erfindungsgemäßer Retraktor 10 in die Operationsöffnung eingesetzt wurde. Die Operationsöffnung wurde durch Auftrennen des Brustbeins in die beiden Teile 70 und 72 gebildet. Die beiden Hälften des Brustbeins bilden einen Teil des Brustkorbs 74. Innerhalb des Brustkorbs 74 sind die beiden Lungenflügel 76 und 78 und das Herz 80 dargestellt. Im unteren Bereich des Brustkorbs 74 ist ein Rückenwirbel 82 dargestellt.

Der Retraktor 10 befindet sich in dieser Darstellung in einem noch nicht gespreizten Zustand. Durch Drehen der Kurbel 22 kann der Haltearm 24 entlang der Schiene 12 in Richtung des Pfeils 84 von dem Haltearm 14 wegbewegt werden. Dadurch wird das Funktionselement 26 von der Valve 16 in Richtung des Pfeils 84 wegbewegt und die Operationsöffnung in Brustkorb 74 des Patienten wird erweitert.

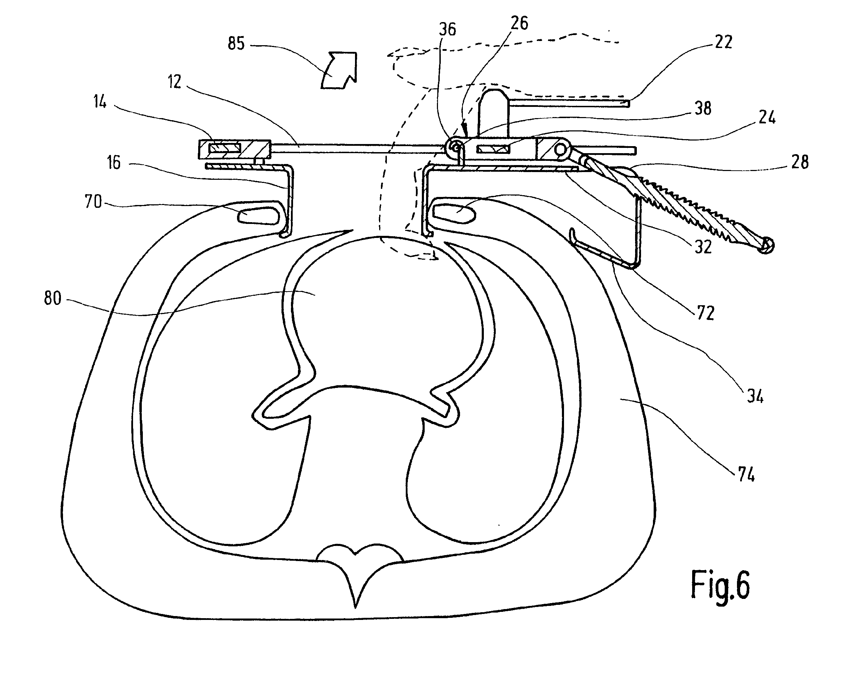

Fig. 6 zeigt eine Darstellung ähnlich der in Fig. 5, wobei durch Betätigen der Kurbel 22, wie zuvor beschrieben, der Haltearm 24 vom Haltearm 14 und damit das Funktionselement 26 von der Valve 16 wegbewegt wurden. Hierbei wird deutlich, dass der Zugang zum Herz 80 bereits erleichtert wurde. Mit gestrichelten Linien ist die Hand eines Operateurs angedeutet, die in die Operationsöffnung eingreift, um das Funktionselement 26 in Richtung des Pfeils 85 anzuheben. Hierbei wird der Schwenkbügel 36 angehoben und das Funktionselement 26 gegenüber dem Schwenkbügel 36 ausgeschwenkt. Der abstützende Abschnitt 34 des Funktionselements 26 bleibt hierbei stets in Kontakt mit der Außenseite des Brustkorbs 74. Beim Anheben überläuft die Rastnase 58 die abgeschrägten Flanken der Zähne 60, 62.

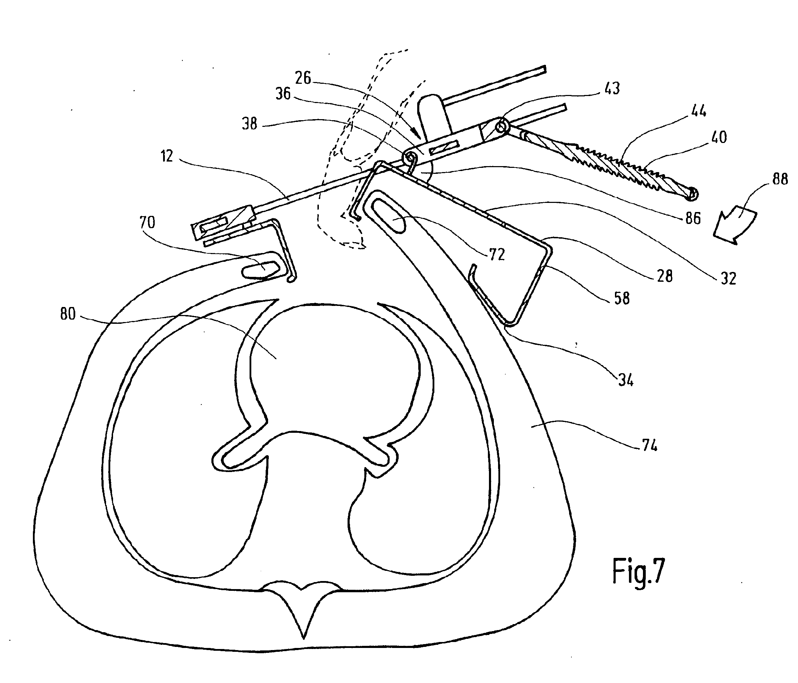

Fig. 7 zeigt einen Schnitt ähnlich zu dem aus Fig. 6, wobei das Funktionselement 26 von der gestrichelt angedeuteten Hand angehoben worden ist. Hierbei ist der Querabschnitt 32 um den Winkel 86 zu dem Schwenkbügel 36 verschwenkt. Dieses Verschwenken erfolgt um den Achszapfen 38. Es wird hierbei außerdem sichtbar, dass die rechte Brustbeinhälfte 72 gegenüber der linken Brustbeinhälfte 70 deutlich angehoben ist und der Zugang zum Herz 80 deutlich verbessert wurde.

Durch den sperrenden Eingriff zwischen Zahnstange 44 und Aussparung 48 verbleibt das Funktionselement 26 und der mit diesem verbundene Haltearm 24 in der angehobenen Stellung. Die enorme Rückstellkraft, die der Brustkorb 74 auf den Riegelmechanismus ausübt, sorgt für feste Verriegelung.

Zum Lösen der Verriegelung wird der Retraktor 10 bzw. das Funktionselement 26 etwas angehoben und das Stellorgan 40 aus der Aussparung 48 angehoben.

Zum erneuten Verriegeln wird das Stellorgan 40 wieder in die Aussparung 48 (siehe Pfeil 88) eingeschwenkt. Diese Bewegung kann durch Federkraft unterstützt werden.

## Patentansprüche

1. Retraktor (10) mit einer Schiene (12), von der ein erster Haltearm (14) abgewinkelt absteht, sowie mit einem zweiten Haltearm (24), der sich etwa parallel zum ersten Haltearm (14) erstreckt und deren Abstand veränderbar ist, und mit an den Haltearmen angebrachten Funktionselementen, von denen zumindest ein Funktionselement (26) einen sich quer zum entsprechenden Haltearm erstreckenden Abschnitt (32) aufweist, der um eine Achse (38), die etwa parallel und im Abstand zum entsprechenden Haltearm (14,24) verläuft verschwenkbar ist, wobei der Abschnitt (32) mit einem Schwenkbügel (36) um die Achse (38) schwenkbar verbunden ist, wobei der Schwenkbügel (36) mit dem entsprechenden Haltearm verbunden ist, **dadurch gekennzeichnet, dass** die jeweilige Schwenkstellung des Funktionselementes (26) relativ zum Schwenkbügel (36) und somit zum entsprechenden Haltearm durch Ergreifen mit einer Hand einstellbar ist und durch einen Riegelmechanismus fixierbar ist.

2. Retraktor nach Anspruch 1, **dadurch gekennzeichnet, dass** der Riegelmechanismus ein Rastenmechanismus ist.

3. Retraktor nach Anspruch 2, **dadurch gekennzeichnet, dass** der Rastenmechanismus eine Zahnstange (44) und eine Rastnase (58) aufweist.

4. Retraktor nach Anspruch 3 **dadurch gekennzeichnet, dass** die Rastnase (58) durch eine Aussparung (48) im Funktionselement (26) gebildet wird.

5. Retraktor nach einem der Ansprüche 3 oder 4, **dadurch gekennzeichnet, dass** die Rastnase (58) an der der Zahnstange (44) zugewandten Seite abgeschrägt ist.

6. Retraktor nach einem der Ansprüche 3 bis 5, **dadurch gekennzeichnet, dass** die Zahnstange (44) sich in etwa im rechten Winkel zur Längsachse des Haltearms (24) von dem Retraktor (10) wegerstreckt.

7. Retraktor nach einem der Ansprüche 3 bis 6, **dadurch gekennzeichnet, dass** die Zahnstange (44) an dem dem Retraktor (10) abgewandten Ende einen T-förmigen Griff (48) aufweist.

8. Retraktor nach einem der Ansprüche 1 bis 7, **dadurch gekennzeichnet, dass** das Funktionselement (26) längs des Haltearms (24) verschiebbar ist.

9. Retraktor nach Anspruch 8, **dadurch gekennzeichnet, dass** das Funktionselement (26) von dem Haltearm (24) abnehmbar ist.

10. Retraktor nach Anspruch 9, **dadurch gekennzeichnet, dass** das Funktionselement (26) an jedem der Haltearme (14, 24) anbringbar ist.

11. Retraktor nach einem der Ansprüche 1 bis 10, **dadurch gekennzeichnet, dass** das Funktionselement (26) an den Flächenbereichen, mit denen es am Rand einer Operationsöffnung oder im Körper anliegt, zumindest teilweise mit einer konturierten Oberfläche versehen ist.

12. Retraktor nach Anspruch 11, **dadurch gekennzeichnet, dass** die Oberflächenstruktur gekreuzt fischgrätenartig ausgebildet ist.

13. Retraktor nach Anspruch 12, **dadurch gekennzeichnet, dass** die Oberflächenstruktur pyramidenförmige Erhebungen mit rautenförmiger Basis aufweist.

14. Retraktor nach einem der Ansprüche 1 bis 12, **dadurch gekennzeichnet, dass** an einem Haltearm (24 das Funktionselement (26) und am anderen eine Valve (16) angebracht ist.

## Claims

1. Retractor (10) with a rail (12) from which a first holding arm (14) protrudes at an angle, and with a second holding arm (24) which extends approximately parallel to the first holding arm (14), a distance between these is modifiable, and with functional elements mounted on the holding arms, of which at least one functional element (26) has a section (32) that extends transversely to the respective arm, and which can be pivoted about an axis (38) which extends roughly parallel and at a distance to the respective arm (14, 24), the section (32) is privotably connected to the axis (38) via a swivel bracket (36), which swivel bracket (36) is in connection with the respective arm, **characterized in that** a particular swivel position of the functional element (26) relatively to the swivel bracket (36) and thus to the respective holding arm can be adjusted by grasping it with one hand and can be fixed by a locking mechanism.

2. Retractor of claim 1, **characterized in that** the locking mechanism is a catch mechanism.

3. Retractor of claim 2, **characterized in that** the catch mechanism has a rack (44) and a locking catch (58).

4. Retractor of claim 3, **characterized in that** the locking catch (58) is formed by a recess (48) within the functional element (26).

5. Retractor of claims 3 or 4, **characterized in that** the locking catch (58) is bevelled on the side facing the rack (44).

6. Retractor of anyone of claims 3 through 5, **characterized in that** the rack (44) extends away from the retractor (10) roughly at a right angle to the longitudinal axis of the holding arm (24).

7. Retractor of anyone of claims 3 through 6, **characterized in that** the rack (44) has a T-shaped handle (48) on the end facing away from the retractor (10).

8. Retractor of anyone of claims 1 through 7, **characterized in that** the functional element (26) can be moved along the holding arm (24).

9. Retractor of claim 8, **characterized in that** the functional element (26) can be removed from the holding arm (24).

10. Retractor of claim 9, **characterized in that** the functional element (26) can be attached to any of the holding arms (14, 24).

11. Retractor of anyone of claims 1 through 10, **characterized in that** the functional element (26) has at least partially a contoured surface on the areas where it is at the edge of an operating window or within the body.

12. Retractor of claim 11, **characterized in that** the structure of the surface has a crossed herringbone pattern.

13. Retractor of claim 12, **characterized in that** the structure of the surface has pyramid-shaped elevations with a lozenge-shaped base.

14. Retractor of anyone of claims 1 through 12, **characterized in that** the functional element (26) is mounted on one holding arm (24), and a valve (16) is mounted on the other.

## Revendications

1. Ecarteur (10) avec une barre ou rail (12) dont fait saillie en faisant un coude un premier bras de retenue (14) ainsi qu'avec un deuxième bras de retenue (24) qui s'étend à peu près parallèlement au premier bras de retenue (14) et dont la distance à celui-ci est variable, et avec des éléments fonctionnels montés sur les bras de retenue, dont au moins un élément fonctionnel (26) présente une section (32) s'étendant perpendiculairement au bras de retenue correspondant, qui est pivotante autour d'un axe (38) qui s'étend à peu près parallèlement au bras de retenue correspondant (14, 24) et à distance de celui-ci, la section (32) étant reliée de manière pivotante autour de l'axe (38) à un étrier pivotant (36), lequel étrier pivotant (36) est relié au bras de retenue correspondant, **caractérisé en ce que** la position de pivotement respective de l'élément fonctionnel (26) par rapport à l'étrier pivotant (36) et donc au bras de retenue correspondant est réglable par saisie avec une main et blocable par un mécanisme de verrouillage.

2. Ecarteur selon la revendication 1, **caractérisé en ce que** le mécanisme de verrouillage est un mécanisme à encliquetage.

3. Ecarteur selon la revendication 2, **caractérisé en ce que** le mécanisme à encliquetage présente une crémaillère (44) et un ergot d'encliquetage (58).

4. Ecarteur selon la revendication 3, **caractérisé en ce que** l'ergot d'encliquetage (58) est formé par un évidement (48) dans l'élément fonctionnel (26).

5. Ecarteur selon l'une des revendications 3 ou 4, **caractérisé en ce que** l'ergot d'encliquetage (58) est biseauté du côté tourné vers la crémaillère (44).

6. Ecarteur selon l'une des revendications 3 à 5, **caractérisé en ce que** la crémaillère (44) s'étend en s'éloignant de l'écarteur (10) à peu près à angle droit par rapport à l'axe longitudinal du bras de retenue (24).

7. Ecarteur selon l'une des revendications 3 à 6, **caractérisé en ce que** la crémaillère (44) présente une poignée en forme de T (48) à son extrémité éloignée de l'écarteur (10).

8. Ecarteur selon l'une des revendications 1 à 7, **caractérisé en ce que** l'élément fonctionnel (26) est déplaçable ou coulissable le long du bras de retenue (24).

9. Ecarteur selon la revendication 8, **caractérisé en ce que** l'élément fonctionnel (26) est séparable du bras de retenue (24).

10. Ecarteur selon la revendication 9, **caractérisé en ce que** l'élément fonctionnel (26) peut être monté sur chacun des bras de retenue (14, 24).

11. Ecarteur selon l'une des revendications 1 à 10, **caractérisé en ce que** l'élément fonctionnel (26) est pourvu au moins partiellement d'une surface profilée sur les zones de surface par lesquelles il s'applique sur le bord d'une ouverture d'opération ou dans le corps.

12. Ecarteur selon la revendication 11, **caractérisé en ce que** la structure de surface est réalisée de manière croisée en arête de poisson.

13. Ecarteur selon la revendication 11, **caractérisé en ce que** la structure de surface présente des surélévations en forme de pyramide à base en losange.

14. Ecarteur selon l'une des revendications 1 à 12, **caractérisé en ce que** l'élément fonctionnel (26) est monté sur un bras de retenue (24) et une valve (16) sur l'autre.
